# EUROPEAN PATENT SPECIFICATION

(11) **EP 2 599 500 B1**
(45) Date of publication and mention of the grant of the patent: **06.04.2016**
(21) Application number: 11812179.7
(22) Date of filing: 15.06.2011
(51) Int. Cl.: A61K 47/34, A61K 9/70, A61K 47/04, A61K 47/32

(54) **MEDICAL ADHESIVE PATCH**
HEFTPFLASTER FÜR MEDIZINISCHE ZWECKE
TIMBRE ADHÉSIF À USAGE MÉDICAL

(30) Priority: 29.07.2010 JP 2010171214
(43) Date of publication of application: 05.06.2013
(73) Proprietor: Hisamitsu Pharmaceutical Co., Inc., Tosu-shi, Saga 841-0017 (JP)
(72) Inventor: TAKADA Yasunori, Tsukuba-shi Ibaraki 305-0856 (JP); SHIMA Takito, Tsukuba-shi Ibaraki 305-0856 (JP); TATEISHI Tetsurou, Tosu-shi Saga 841-0017 (JP); NISHIHARA Tsuguki, Tokyo 110-8560 (JP); YOSHIDA Chiaki, Tokyo 110-8560 (JP); MATSUSHIMA Atsushi, Tokyo 110-8560 (JP); TAKAMIYA Tsuyoshi, Ogori-shi Fukuoka 838-0112 (JP)
(74) Representative: Prüfer & Partner mbB Patentanwälte · Rechtsanwälte
(86) International application number: PCT/JP2011/063666
(87) International publication number: WO 2012/014586

(56) References cited:
- WO-A1-93/11938
- WO-A1-2007/023791
- GB-A- 2 452 086
- GB-A- 2 457 294
- JP-A- 2003 093 434
- JP-A- 2003 136 645
- JP-A- 2006 016 382
- JP-A- 2009 536 647

## Description

### BACKGROUND OF THE INVENTION

### Field of the Invention

The present invention relates to a medical adhesive patch and particularly to a medical adhesive patch which is used for percutaneous administration of a medicine.

### Description of Related Art

A tape in which an adhesive layer is formed on one surface of a sheet-shaped or film-shaped support is widely used for various purposes such as in the medical or industrial fields. In the medical field, a percutaneous administration of medicine is performed using a medical adhesive patch which mixes a medicine into an adhesive layer. The administration of a medicine with the medical adhesive patch is advantageous since it is only slightly invasive to a patient, and the study thereof has progressed in order to broaden the applicable range of medicines.

In addition to an adhesive material, a medicine or a plasticizer is mixed into the adhesive layer of the medical adhesive patch. Since there is a concern of a negative effect due to adsorption of a plasticizer depending on the material of a support, it is preferable that at least a surface of the support which comes in contact with the adhesive layer has barrier properties. In addition, when the medicine is adsorbed into the support, there is a concern that necessary amounts of the medicine cannot be administered, so it is necessary for the support to obtain a barrier property.

A gas barrier film disclosed in Patent Document 1 (Japanese Unexamined Patent Application, First Publication No. 2003-136645) has been known as a film material having a barrier property. In the gas barrier film, a barrier coating layer is formed by applying a barrier coating material which is obtained by mixing montmorillonite which is a layered inorganic compound and a water-soluble polymer compound, on one surface of a base material film made of plastic.

In addition, Patent Document 2 (Japanese Unexamined Patent Application, First Publication No. H08-127531) discloses a film-shaped support for percutaneous administration of a medicine including a barrier film formed of polyethylene terephthalate (PET) or the like. Patent Document 3 (Japanese Unexamined Patent Application, First Publication No. 2009-249298) discloses a film-shaped support for an adhesive patch having a barrier property by forming a vapor-deposited layer formed of aluminum or the like on the support. Patent Document 4 (Japanese Unexamined Patent Application, First Publication No. 2009-173626) discloses a support for an adhesive patch obtained by laminating an elastic base film layer and a polyester resin film layer which includes a groove, the width of which changes according to elongation of the base film.

On the other hand, in some cases, the medical adhesive patch is attached to a patient in a state where a length or an area thereof is increased by the medical adhesive patch elongating from an initial state, and the medical adhesive patch is elongated due to motions of a patient after being attached, on a part where it bends or stretches, such as a trunk, an elbow, or a knee. Accordingly, a support having excellent flexibility is preferable for the support used for the medical adhesive patch.

A gas barrier film disclosed in Patent Document 1 is conceived to be used mainly for packaging materials of food products, electronic components, and the like, and as materials of a base film, a biaxially-drawn polyester film, a polypropylene film and the like are disclosed, however, since these materials may not have high flexibility, the films may not have a preferable configuration as they are, as a support film for a medical adhesive patch which is used in the environment described above.

Herein, the inventors found the following problems when configuring a medical adhesive patch by selecting a material with excellent flexibility, and applying the material to a support.

That is, it is possible to prepare a medical adhesive patch using a support formed of a material with excellent flexibility, however, if the medical adhesive patch is elongated when being attached or after being attached, a barrier layer cannot sufficiently respond to a change in shape of the support due to the elongation, and cracks or the like are generated on the barrier layer, in some cases.

If cracks or the like are generated on the barrier layer, the barrier properties of the barrier layer are degraded, and if the cracks or the like pass through in a thickness direction of the barrier layer, the barrier properties are lost. As a result, there is a problem with a case in which the barrier layer does not have an enough performance in use and the negative effect to the support due to the plasticizer described above or the degradation of the amount medicine administered cannot be sufficiently suppressed.

On the other hand, as disclosed in Patent Document 2, in a configuration including a barrier film formed of PET and the like, there is a problem in that flexibility of the support is not sufficiently realized due to PET and the like which have low flexibility.

In addition, in the barrier layer formed of the vapor-deposited layer as disclosed in Patent Document 3, cracks and the like are easier to occur, such that the barrier layer is not suitable for a medical adhesive patch. Additional prior art includes WO 93/11938.

### SUMMARY OF THE INVENTION

The present invention has been made to address the aforementioned problems, and aims at providing a medical adhesive patch which maintains an excellent barrier property even with elongation.

A medical adhesive patch of the present invention, includes:a support film comprising a barrier layer containing polyvinyl alcohol with a degree of saponification in a range equal to or more than 70% and equal to or less than 95.5% and montmorillonite, the barrier layer being laminated on one surface of a support including polyurethane; and an adhesive layer containing a medicine, a plasticizer, and a rubber-based polymer, being laminated on a barrier layer of the support film, wherein a percentage content of montmorillonite in the barrier layer is equal to or more than 10 percent by weight and equal to or less than 22 percent by weight, and the plasticizer is isopropyl myristate or glyceryl monoisostearate.

According to the medical adhesive patch of the present invention, it is possible to maintain an excellent barrier property of the adhesive patch even with elongation of the adhesive patch during or after attachment.

### BRIEF DESCRIPTION OF THE DRAWINGS

FIG 1 is a cross-sectional view of a medical adhesive patch of an embodiment of the present invention in a thickness direction.
FIG. 2 is a view showing a procedure of an experiment for checking a suitable range of the amount of montmorillonite in a barrier layer.
FIG 3 is a view showing a procedure of the same experiment.
FIG 4 is a view showing a procedure of the same experiment.
FIG. 5 is a view showing a procedure of the same experiment.
FIG 6 is an optical micrograph of a barrier layer after an elongation operation with an elongation rate of 20% with respect to an evaluated piece with an amount of montmorillonite in a barrier layer of 10 wt%.
FIG. 7 is an optical micrograph of a barrier layer after an elongation operation with an elongation rate of 20% with respect to an evaluated piece with an amount of montmorillonite in a barrier layer of 18 wt%.
FIG. 8 is an optical micrograph of a barrier layer after an elongation operation with an elongation rate of 20% with respect to an evaluated piece with an amount of montmorillonite in a barrier layer of 25 wt%.
FIG. 9 is an optical micrograph of a barrier layer after an elongation operation with an elongation rate of 20% with respect to an evaluated piece with an amount of montmorillonite in a barrier layer of 30 wt%.
FIG. 10 is a graph showing a relationship between the amount of montmorillonite and the modulus value of a support film.
FIG. 11 is a view showing a procedure of an experiment for checking for a relationship between the degree of saponification of a water-soluble polymer compound and the adhesiveness of support-barrier layer.
FIG. 12 is a view showing a procedure of the same experiment.
FIG. 13 is a view showing a procedure of the same experiment.
FIG. 14 is a view showing a procedure of the same experiment.
FIG. 15 is a view showing a procedure of the same experiment.

### DETAILED DESCRIPTION OF THE INVENTION

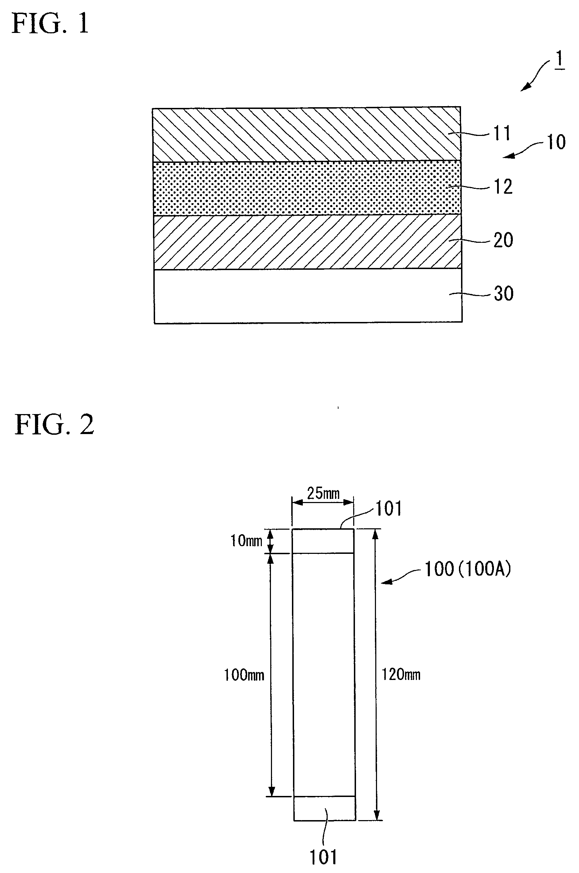

Hereinafter, a medical adhesive patch of an embodiment of the present invention will be described referring to FIGS. 1 to 15. FIG. 1 is a cross-sectional view of a medical adhesive patch 1 of the embodiment in a thickness direction. The medical adhesive patch 1 includes a support film 10, an adhesive layer 20 which is formed on one surface of the support film 10 and contains a medicine, and a peel-off member 30 which covers the adhesive layer.

The support film 10 includes a support 11 which is formed of polyurethane or which includes polyurethane and is formed in a film shape or a sheet shape, and a barrier layer 12 which is formed on one surface of the support 11.

The support 11 includes flexibility and can be elongated by a predetermined maximum elongation rate increasing equal to or more than 10 percent (%) in length A detailed value of the maximum elongation rate may be suitably set based on the purpose or attachment portion of the medical adhesive patch 1. In the embodiment, the polyurethane which forms the support 11 is not particularly limited, polyurethane used in a polyurethane film of the related art can be used, and it can be suitably selected on purpose. For example, polyether-based polyurethane, polyester-based polyurethane, polycarbonate-based polyurethane may be used. For the purpose of water resistance, polyether-based polyurethane or polycarbonate-based polyurethane is preferable.

In addition, the type of isocyanate forming urethane bond is not particularly limited, and a yellowing type, or a non-yellowing type can be used, and a suitable selection can be made according to the purpose, storing period or method in usage, types of used plasticizer and the like.

The thickness of the support 11 is 10 micrometers (µm) to 200 µm, and is preferably equal to or more than 15 µm and equal to or less than 100 µm. When the thickness is less than 10 µm, the support is difficult to handle as it is too thin, and when the thickness is more than 200 µm, the flexibility is reduced such that original flexibility is not sufficiently exhibited.

The support 11 can include a film called a release film having a peel-off property. When the thickness of the support 11 is thin, since the support is elongated in a step of applying the barrier layer 12, if manufacturing in a state that the release film and the support (for example, polyurethane as the support) are laminated, it is possible to easily process while suppressing the elongation of the support. In addition, since the rigidity of the tape is reinforced by the release film after processing the support 11 on the tape, the handleability of the tape is improved. In the case of using the release film, the tape can be adhered to an object and peel it from the support, such that the support 11 after the peel-off exhibits original flexibility.

The material of the release film is not particularly limited; however, generally, a material which can be peeled off without performing elongation or contraction, such as a silicon-treated PET film, a polyolefin film having an excellent peel-off property, an aggregate such as paper or polyethylene, or the like, can be used.

The barrier layer 12 is formed to include montmorillonite which is a layered inorganic compound and polyvinyl alcohol (PVA) which is a water-soluble polymer compound.

Mineralogically, the montmorillonite is a dioctahedral type water bearing layered silicate mineral and is ideally expressed as the following equation.

(Al_{2-y}Mg_{y})Si₄O₁₀(OH)₂ · (M⁺,M_{1/2}²⁺)y · nH₂O

Herein, y = 0.2 to 0.6; M is an exchangeable cation such as Na, K, Ca, Mg, or H; and n is the amount of interlayer water.

A crystal structure of montmorillonite forms a layered structure which includes three layers formed of two tetrahedral sheets and one octahedral sheet as a base. The cation of the tetrahedral sheets is only Si, and some of the cations Al of the octahedral sheet are substituted with Mg. Accordingly, a unit crystal layer takes on a negative electric charge, and cations such as Na⁺, K⁺, Ca²⁺, Mg²⁺, H⁺, and the like enter and compensate for electric charges between crystal layers so as to balance with the negative electric charge. In the present invention, there are no particular limitations on the types of cations that can be used.

After the montmorillonite is added to and dispersed in a water solution obtained by melting PVA in water, the barrier layer 12 can be formed by applying a barrier coating material obtained by adding and adjusting lower alcohol to the water solution containing the montmorillonite and the melting PVA with a gravure coating method or a roll coating method. If necessary, an anchor coating layer may be formed on the support 11 and the barrier layer 12 may be formed through the anchor coating layer. In the same manner, the barrier layer 12 may be formed after being subjected to a surface treatment on the support 11. As the surface treatment, a corona discharge treatment or a plasma discharge treatment is preferable. From the above, the corona discharge treatment is more preferable from the viewpoint of general versatility or handleability.

The amount of montmorillonite in the barrier layer 12 is in a range specified above. A detailed description will be provided later, however, if the amount is less, it is difficult to secure a sufficient barrier property. On the other hand, if the amount exceeds 22 wt%, an effect caused by the montmorillonite to the physical property of the barrier layer 12 becomes too much, and as a result, sufficient response to shape change of the support due to the elongation cannot be performed, and cracks or the like may be easily generated.

In addition, when a patient has a bath in a state where the medical adhesive patch 1 is attached, if the adhesiveness of the support 11 and the barrier layer 12 is not sufficient due to the medical adhesive patch 1 being attached for a long period, in some cases, the support 11 is peeled off from the barrier layer 12, and separated from the adhesive layer 20. The PVA is a polymer compound which is obtained by saponification of polyvinyl acetate (alkaline hydrolysis treatment) and includes a hydroxyl group; however, in the medical adhesive patch 1 of the embodiment, in order to maintain excellent adhesiveness of the barrier layer 12 and the support 11 to prevent the situation described above, the degree of saponification of PVA is in a range equal to or more than 70% and equal to or less than 95.5%. A detailed description thereof will be also provided later, however, if the degree of saponification exceeds 95.5%, the adhesiveness with the support 11 is degraded, and if the degree of saponification is less than 70%, the barrier layer 12 becomes easy to melt in water, and as a result, the water resistance of the medical adhesive patch 1 is degraded.

The adhesive layer 20 is configured by mixing a plasticizer and a medicine to a base material having an adhesive property and is formed by applying the mixtures of the plasticizer and the medicine or the like thereof on the barrier layer 12 and a surface opposite to the support 11.

As an adhesive used in the adhesive layer 20, an adhesive which includes rubber-based polymers as a base can be used.

As the rubber-based polymers, styrene - isoprene - styrene block copolymer (hereinafter, referred to as SIS), isoprene rubber, polyisobutylene (hereinafter, referred to as PIB), styrene-butadiene-styrene block copolymer (hereinafter, referred to as SBS), styrene-butadiene rubber (hereinafter, referred to as SBR), polysiloxane can be used.

The plasticizer is as specified above.

The mixing amount of the plasticizer of the adhesive layer 20 is from 10 to 70 wt%, preferably from 10 to 60 wt%, and more preferably from 10 to 50 wt%.

Furthermore, for improving adhesiveness, various tackifier may be mixed For example, a rosin derivative (for example, rosin, glycerol ester of rosin, hydrogenated rosin, glycerin ester of hydrogenated rosin, pentaerythritol ester of rosin), alicyclic saturated hydrocarbon resin (for example, product name: ARKON (tradename) P100 manufactured by Arakawa Chemical Industries, Ltd.) one aliphatic hydrocarbon resin (for example, product name: Quintone (tradename) B170 manufactured by ZEON Corporation), terpene resin (for example, product name: Clearon (tradename) P-125 manufactured by YASUHARA CHEMICAL Co., Ltd.), maleic acid resin, is used. Among them, glycerin ester of hydrogenated rosin, alicyclic saturated hydrocarbon resin, aliphatic hydrocarbon resin, and terpene resin are particularly preferable.

The mixing amount of a tackifier of the adhesive layer 20 is from 5 to 70 wt%, preferably 5 to 60 wt%, and more preferably 10 to 50 wt%.

Furthermore, an absorption promoter may be mixed for absorption promotion of a medicine into a body.

As the absorption promoter, fatty acids having carbon chain numbers of 6 to 20, fatty alcohols, fatty acid esters, amides, or ethers, aromatic organic acids, aromatic alcohols, aromatic organic acid ester or ethers (the above components may be saturated or unsaturated, or may be cyclic, linear, or branched), lactic acid esters, acetic acid esters, a monoterpene compound, a sesquiterpene compound, Azone, an Azone derivative, pyrothiodecane, glycerin fatty acid esters, propylene glycol fatty acid esters, sorbitan fatty acid esters (Span based), polysorbates (Tween based), polyethylene glycol fatty acid esters, polyoxyethylene cured castor oil based (HCO based), polyoxyethylene alkyl ethers, sucrose fatty acid esters, vegetable oil, is used.

A mixing amount of the absorption promoter of the adhesive layer 20 is from 0.01 to 20 wt%, preferably 0.05 to 10 wt%, and more preferably 0.01 to 5 wt%.

In addition, the absorption promoter is an absorption promoter which includes a part of plastic acting, and in the present invention, such absorption promoter can be used as a plasticizer. Furthermore, the material described above exemplified as the absorption promoter may be used as a softener, a solubilizer, a solubilizing agent, or a solubilizer.

In addition, antioxidants, fillers, cross-linking agents, ultraviolet absorbers, preservatives or the like may be suitably added if necessary.

The medicine which exhibits a medicinal effect by being mixed to the adhesive layer 20 basically has no limitation, and various medicines can be applied thereto. In addition, a plurality of medicines may be mixed in combination.

It is not particularly limited thereto, and for example, hypnotic and sedatives (flurazepam hydrochloride, rirumazahon hydrochloride, phenobarbital, amobarbital; ; antipyretic analgesic (butorphanol tartrate, citrate perisoxal, acetaminophen, mefenamic acid, diclofenac sodium, aspirin, alclofenac, ketoprofen, flurbiprofen, naproxen, piroxicam, pentazocine, indomethacin, glycol salicylate, aminopyrine, loxoprofen); a steroidal anti-inflammatory drug (hydrocortisone, prednisolone, dexamethasone, betamethasone); stimulants and excitants (methamphetamine hydrochloride, methylphenidate hydrochloride); psychotropic agents (imipuran hydrochloride, diazepam, sertraline hydrochloride, fluvoxamine maleate, paroxetine hydrochloride, citalopram hydrobromide, fluoxetine hydrochloride, alprazolam, haloperidol, clomipramine, amitriptyline, desipramine, amoxapine, maprotiline, mianserin, setiptiline, trazodon, rohepuramin, milnacipran, duloxetine, venlafaxine, chlorpromazine hydrochloride, thioridazine, diazepam, meprobamate, etizolam, risperidone, mirtazapine); a hormone drug (estradiol, estriol, progesterone, norethisterone acetate, acetic acid meteronon, testosterone,); local anesthetics (lidocaine hydrochloride, procaine hydrochloride, tetracaine hydrochloride, dibucaine hydrochloride, propitocaine hydrochloride); agents affecting urinary organs (oxybutynin hydrochloride, tamsulosin hydrochloride, propiverine hydrochloride, tolterodine tartrate, fesoterojin, imidafenacin); muscle relaxant suxamethonium (tizanidine hydrochloride, eperisone hydrochloride, pridinol mesylate, hydrochloride suxamethonium); agents affecting genital organs (ritodrine hydrochloride, meluadrine tartrate); antiepileptics (sodium valproate, clonazepam, carbamazepine); a medicine for autonomic nerves (carpronium chloride, neostigmine bromide, bethanechol chloride); antiparkinsonian drugs (pergolide mesylate, bromocriptine mesylate, trihexyphenidyl hydrochloride, amantadine hydrochloride, ropinirole hydrochloride, talipexole hydrochloride, cabergoline, droxidopa, piperiden, selegiline hydrochloride, or the like); a diuretic (hydroflumethiazide, furosemide); a respiratory stimulant (lobeline hydrochloride, dimorpholamine, naloxone hydrochloride); an antimigraine agent (dihydroergotamine mesylate, sumatriptan, ergotamine tartrate, flunarizine hydrochloride, cyproheptadine hydrochloride); an antihistamine (clemastine fumarate, diphenhydramine tannate, chlorpheniramine maleate, diphenylpyraline hydrochloride, promethazine); bronchodilator agents (tulobuterol hydrochloride, procaterol hydrochloride, salbutamol sulfate, clenbuterol hydrochloride, fenoterol hydrobromide, terbutaline sulfate, isoprenaline sulfate, formoterol fumarate); cardiotonic agents (isoprenaline hydrochloride, dopamine hydrochloride); coronary vasodilators (diltiazem hydrochloride, verapamil hydrochloride, isosorbide dinitrate, nitroglycerin, nicorandil); peripheral vasodilators (nicametate citrate, tolazoline hydrochloride); smoking-cessation aid (nicotine ); agents for circulatory organs (flunarizine hydrochloride, nicardipine hydrochloride, nitrendipine, nisoldipine, felodipine, amlodipine besylate, nifedipine, nilvadipine, manidipine hydrochloride, benidipine hydrochloride, enalapril maleate, temocapril hydrochloride, alacepril, imidapril hydrochloride, cilazapril, lisinopril, captopril, trandolapril, perindopril erbumine, atenolol, pindolol, bisoprolol fumarate, metoprolol tartrate, betaxolol hydrochloride, timolol maleate, bopindolol malonate, nipradilol, arotinolol hydrochloride, celiprolol hydrocloride, carvedilol, amosulalol hydrochloride, carteolol hydrochloride, bevantolol hydrochloride, terazosin hydrochloride, bunazosin hydrochloride, prazosin hydrochloride, doxazosin mesylate, valsartan, candesartan cilexetil, losartan potassium, clonidine hydrochloride, guanfacine hydrochloride, guanabenz acetate); antiarrhythmic agents (propranolol hydrochloride, alprenolol hydrochloride, procainamide hydrochloride, mexiletine hydrochloride, nadolol, disopyramide); antineoplastic ulcerogenic drugs (cyclophosphamide, fluorouracil, tegafur, procarbazine hydrochloride, ranimustine, irinotecan hydrochloride, fururijin); antilipemic drugs (pravastatin, simvastatin, bezafibrate, probucol); hypoglycaemic drugs (glibenclamide, chlorpropamide, tolbutamide, glymidine sodium, glybuzole, buformine hydrochloride): antiulcer drugs (proglumide, cetraxate hydrochloride, spizofurone, cimetidine, glycopyrronium bromide); cholagogue (ursodesoxycholic acid, osalmid); enterokinesis improving agents (domperidone, cisapride); agents for liver disease (thiopronine); antiallergic agents (ketotifen fumarate, azelastine hydrochloride); antiviral drugs (acyclovir); antimotionsickness agents (betahistine mesylate, difenidol hydrochloride); antibiotics (cephaloridine, cefdinir, cefpodoxime proxetil, cefaclor, clarithromycin, erythromycin, methyl erythromycin, kanamycin sulfate, cycloserine, tetracycline, benzylpenicillin potassium, potassium propicillin, cloxacillin sodium, ampicillin sodium, bacampicillin hydrochloride, carbenicillin sodium, chloramphenicol); agents for habitual intoxication (cyanamide); anoretics (mazindol); chemotherapeutic agents (isoniazid, ethionamide, pyrazinamide); blood coagulation accelerants (ticlopidine hydrochloride, warfarin potassium); anti-alzheimers' agents (physostigmine, donepezil hydrochloride, tacrine, arecoline, xanomeline); serotonin receptor antagonistic antiemetics (ondansetron hydrochloride, granisetron hydrochloride, ramosetron hydrochloride, azasetron hydrochloride, palonosetron); gout remedies (colchicine, probenecid, sulfinpyrazone); illegal analgesics (fentanyl citrate, morphine sulfate, morphine hydrochloride, codeine phosphate, cocaine hydrochloride, pethidine hydrochloride); is used.

In particular, since the medical adhesive patch of the present invention can be attached to the skin for a long period, for example, for about one week, it is not necessary to change the medical adhesive patch every day and the workload of a patient or a caretaker is reduced, and accordingly, the correct administration is improved.

In the medical adhesive patch 1 of the embodiment, by suitably setting the amount of montmorillonite of the barrier layer 12 in the range described above while considering types of plasticizers or medicines, the transition of plasticizers or medicines to the support 11 from the barrier layer12 is suitably suppressed.

Generally, polyurethane configuring the support 11 easily performs adsorption of the plasticizers or medicines, and in that case, there is a concern that the problems described above occur; however, in the medical adhesive patch 1, the barrier property of the barrier layer 12 is suitably maintained even in non-elongation time or with an elongation rate of 20% (length after the elongation indicates an increase of 20%) of the support 11. As a result, it is possible to suitably prevent the problems described above, even when the medical adhesive patch 1 is used while being attached to an object, not only when being stored before use. In addition, the amount of the montmorillonite can be easily set in detail by a preliminary experiment using the plasticizer or the medicine to be used. The relationship between the barrier property with respect to a part of the plasticizer and the medicine and the amount of montmorillonite will be described later.

The peel-off member 30 is a member to protect an adhesive surface of the adhesive layer 20 until the adhesion to a patient, and various types of well-known release paper can be suitably used. In addition, when the medical adhesive patch 1 is rolled up on a core, the peel-off member 30 may not be prepared.

Next, an experiment and a result thereof which are performed for reviewing a suitable range of the amount of the montmorillonite (hereinafter, referred to as "MN", in some cases) of the barrier layer 12 and a suitable range of the degree of saponification of the PVA will be described.

### (Experiment 1 Evaluation of Relationship between Barrier Property and Amount of MN at the Time of Elongation: Evaluation with Swelling of Support as Index)

### (1-1 Preparation of Sample)

As a support, a material prepared by polyether-based polyurethane having a thickness of 20 µm was used. A barrier layer was formed by uniformly applying 1.0 g/m² of a barrier coating material which was obtained by mixing MN and PVA (with a degree of saponification of 80%) on one surface of the support. By setting this as a basic configuration, 8 stages of the amount of MN of the barrier layer were 1 wt%, 2 wt%, 10 wt%, 18 wt%, 22 wt%, 25 wt%, 30 wt%, and 37 wt%, and 8 types of samples of support films as materials of medical adhesive patches were prepared.

### (1-2 Experiment Procedure)

The prepared 8 types of samples 100 were cut to be a size of 25 millimeters (mm) X 120 mm as shown in FIG. 2, and in order to perform easy operation with a tensile tester, a sheet 101 prepared by polyethylene terephthalate (PET) having a thickness of 50 µm was attached to the both surfaces of both ends in a longitudinal direction with double-sided tape and an evaluation piece 100A was prepared. A length of the evaluation piece 100A of the sheet 101 in the longitudinal direction was 10 mm, and in each evaluation piece 100A, a length of a portion which is not covered with the sheet 101 in the longitudinal direction was 100 mm.

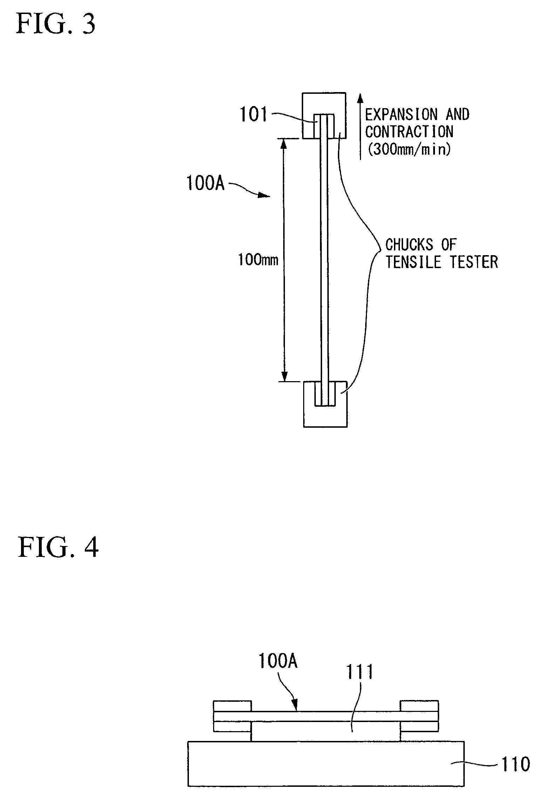

Both ends of the evaluation piece 100A reinforced by the sheet 101 were fixed to the chuck unit of the tensile tester, and as shown in FIG. 3, the portion not covered with the sheet 101 was elongated to reach a predetermined elongation rate with an with the sheet 101 was elongated to reach a predetermined elongation rate with an elongation speed of 300 mm per minute (mm/min). 5 stages of elongation rate were 0%, 5%, 10%, 20%, and 30%.

After completing the elongation operation, the evaluation piece 100A was taken off from the tensile tester, and as shown in FIG. 4, each evaluation piece 100A was fixed onto a black acrylic plate 110 obtained by attaching a PET sheet 111 obtained by applying silicon on the surface thereof, with a barrier layer to be on the upper side. At that time, the preparation is performed so that air does not enter between the evaluation piece 100A and the PET sheet 111.

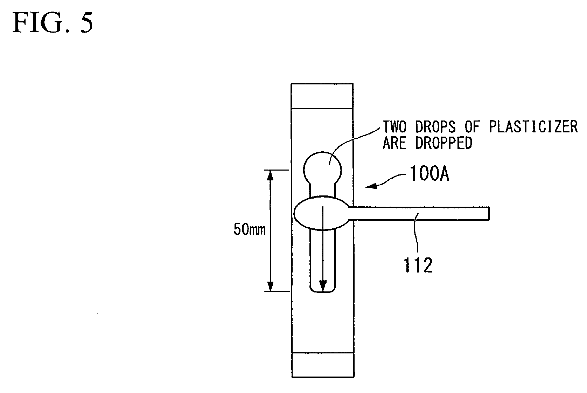

After attaching to the acrylic plate 110, as shown in FIG. 5, two drops (approximately 0.08 grams) of plasticizers was put on each evaluation piece 100 by a dropper, and the evaluation piece was expanded to have a length of 50 mm by using a cotton swab 112. As plasticizers, four types of isopropyl myristate (IPM) (invention), triacetin (TA) (comparison), glyceryl monoisostearate (MGIS) (invention), and sorbitan monooleate (SMO) (comparison) were used. After being left for 30 minutes at a room temperature, the plasticizers were wiped off and the degree of swelling of the support was visually evaluated. As an index, wrinkles of the support generated due to the swelling were used (two stages of: wrinkles due to swelling are not recognized: Good, and wrinkles due to swelling are recognized: Bad).

The IPM, the TA, and the SMO were evaluated using the evaluation pieces 100A having an amount of MN of 1 wt%, 10 wt%, 18 wt%, 22 wt%, 25 wt%, 30 wt%, and 37 wt%, and MGIS was evaluated using the evaluation pieces 100A having an amount of MN of 2 wt%, 10 wt%, and 22 wt%.

### (1-3 Result)

The result is shown in Table 1. When the amount of MN is equal to or lessthan 22 wt% with the IPM, TA, and MGIS, the swelling of the support with all

On the other hand, with the SMO, the swelling is recognized on the support regardless of the amount of MN and the elongation rates, and it was considered that the SMO is not preferable as the plasticizer to be used for a film material of the present invention, in some cases. Solubility parameters (SP value based on Fedors method) of each plasticizer used in the experiment were 8.5 for IPM, 10.2 for TA, 10.7 for MGIS, and 11.76 for SMO, and it is assumed that the plasticizer having a low SP value tends to be preferable.

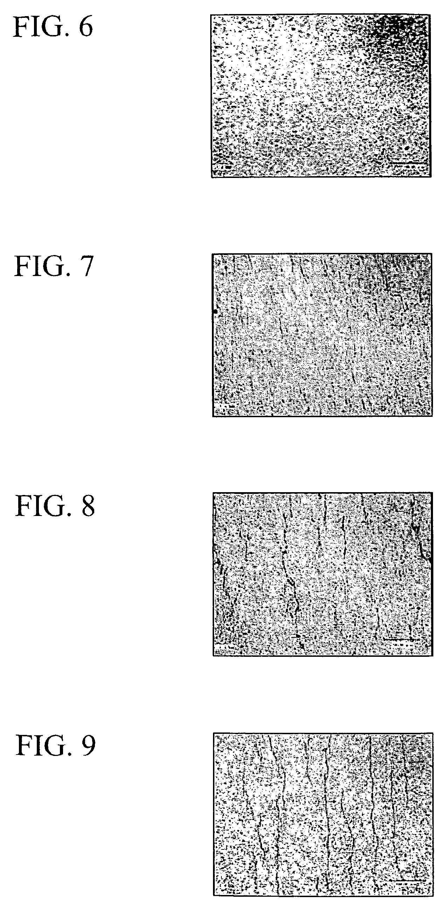

FIGS. 6 to 9 are optical micrographs of the support film after performing the elongation operations with an elongation rate of 20% with respect to the evaluated pieces with an amount of MN of 10 wt%, 18 wt%, 25 wt%, and 30 wt%. In a case of 10 wt% and 18 wt% of the MN, significant changes on the external portion are not recognized, however, in a case of 25 wt% and 30 wt% of the MN, wrinkles due to the swelling are recognized.

**[Table 1]**

| Sample configuration | | | Evaluation | | | | | | | | | | | | | | | | | | | |
|---|---|---|---|---|---|---|---|---|---|---|---|---|---|---|---|---|---|---|---|---|---|---|
| Support | Barrier layer formula | | IPM | | | | | TA | | | | | MGIS | | | | | SMO | | | | |
| | Degree of saponification of PVA | Amount of MN | Elongation rate (%) | | | | | Elongation rate (%) | | | | | Elongation rate (%) | | | | | Elongation rate (%) | | | | |
| | % | wt% | 0 | 5 | 10 | 20 | 30 | 0 | 5 | 10 | 20 | 30 | 0 | 5 | 10 | 20 | 30 | 0 | 5 | 10 | 20 | 30 |
| Ethers 20 µm | 80 | 1 | Good | Good | Good | Good | Good | Good | Good | Good | Good | Good | None | None | None | None | None | Bad | Bad | Bad | Bad | Bad |
| | | 2 | None | None | None | None | None | None | None | None | None | None | Good | Good | Good | Good | Good | None | None | None | None | None |
| | | 10 | Good | Good | Good | Good | Good | Good | Good | Good | Good | Good | Good | Good | .Good | Good | Good | Bad | Bad | Bad | Bad | Bad |
| | | 18 | Good | Good | Good | Good | Good | Good | Good | Good | Good | Good | None | None | None | None | None | Bad | Bad | Bad | Bad | Bad |
| | | 22 | Good | Good | Good | Good | Good | Good | Good | Good | Good | Good | Good | Good | Good | Good | Good | Bad | Bad | Bad | Bad | Bad |
| | | 25 | Good | Good | Bad | Bad | Bad | Good | Bad | Bad | Bad | Bad | None | None | None | None | None | Bad | Bad | Bad | Bad | Bad |
| | | 30 | Good | Bad | Bad | Bad | Bad | Good | Bad | Bad | Bad | Bad | None | None | None | None | None | Bad | Bad | Bad | Bad | Bad |
| | | 37 | Bad | Bad | Bad | Bad | Bad | Bad | Bad | Bad | Bad | Bad | None | None | None | None | None | Bad | Bad | Bad | Bad | Bad |

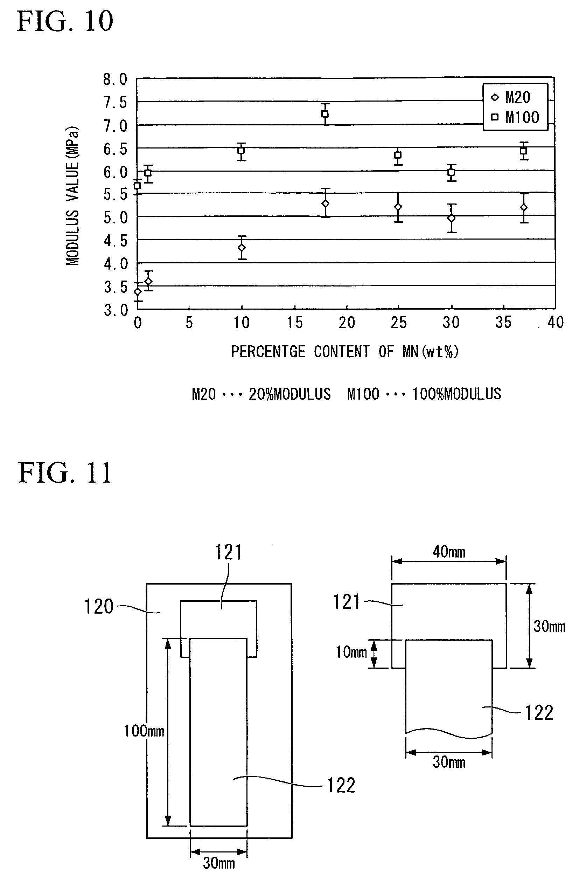

In addition, in each sample, when evaluating modulus after the elongation operation based on a test method of a polyurethane-based thermoplastic elastomer (JIS K 7311), as shown in FIG. 10, the modulus was equal to or less than 8 Mega Pascals (MPa) for all samples, and excellent flexibility was shown. Accordingly, it was evaluated that the barrier layer does not negatively affect the flexibility of the support film.

### (Experiment 2 Evaluation of Relationship between Barrier Property and Amount of MN: Evaluation Using Adhesive Layer Containing Plasticizer)

### (2-1 Preparation of Sample)

The same material as Experiment 1 was used as a support, and a barrier layer was formed by uniformly applying 1.0 g/m² of a barrier coating material which was obtained by mixing MN and PVA (with a degree of saponification of 80%) on one surface of the support. 9 stages of the amount of MN of the barrier layer are 1 wt%, 2 wt%, 4 wt%, 10 wt%, 18 wt%, 22 wt%, 25 wt%, 30 wt%, and 37 wt%, and 9 types of samples of support films are prepared.

In addition, an adhesive layer (applied amount of adhesive layer: 100 g/m²) including a base material and a plasticizer was formed on the barrier layer. Two types of rubber base material and an acrylic base material were used as the base material, and a total of 5 types of adhesive layer materials were prepared by combining each base material with a plurality of types of plasticizers. The adhesive layer was formed on each sample using each adhesive layer material, and samples of medical adhesive patches not containing medicines were prepared by covering the adhesive layer with a peel-off member. The combinations of the base material and the plasticizer for each adhesive layer material are as follows (% of the plasticizer indicates the amount). Rubber base material (IPM 20%, MGIS 10%, SMO 10%, and SMO 20%) and the acrylic base material (IPM 20%, TA 10%, MGIS 10%, and SMO10%)

### (2-2 Experiment Procedure)

### a. Stability Test for Non-Elongation Time

A medical adhesive patch sample obtained by cutting to be 10 square centimeters was stored at 60°C for 1 week without performing an elongation operation.

### b. Stability Test for Elongation Time

A tape sample obtained by cutting to have a width of 30 mm and a length of 50 mm was stored at 60°C for three days after removing the peel-off member and performing the elongation operation with an elongation rate of 20% in a length direction once.

In all cases, in each tape sample after storing, in the same manner as Experiment 1, the barrier property was evaluated by the generation of wrinkles of the support. The evaluation of Experiment 2 was set as three stages. Wrinkles due to the swelling are not recognized: Excellent, slight wrinkles due to the swelling are recognized, but they do not affect the quality: Good, and wrinkles due to the swelling are recognized and the support cannot be used: Bad.

### (2-3 Experiment Results)

The result is shown in Table 2. It is shown that when the amounts of the MN are 2 wt%, 10 wt%, and 18 wt%, in any of the non-elongation time and the 20% elongation time, wrinkles are not recognized in the support and the barrier property is excellently maintained.

In addition, for the SMO which was considered to be not preferable for some times in Experiment 1, it was evaluated that the transition of the plasticizers to the support can be sufficiently suppressed by suitably setting the amount of MN of the barrier layer or the amount of the plasticizers of the adhesive layer.

**[Table 2]**

| Amount of MN (wt%) | Rubber based material | | | | | | | | Acrylic based material | | | | | | | |
|---|---|---|---|---|---|---|---|---|---|---|---|---|---|---|---|---|
| | IPM 20% | | MGIS 10% | | SMO 10% | | ISMO 20% | | IPM 20% | | TA 10% | | MGIS 10% | | SMO 10% | |
| | Non elongation | Elongation rate 20% | Non elongation | Elongation rate 20% | Non elongation | Elongation rate 20% | Non elongation | Elongation rate 20% | Non elongation | Elongation rate 20% | Non elongation | Elongation rate 20% | Non elongation | Elongation rate 20% | Non elongation | Elongation rate 20% |
| 1 | Bad | Bad | Bad | Bad | Bad | Bad | Bad | Bad | Bad | Excellent | Bad | Bad | Bad | Bad | Bad | Bad |
| 2 | Excellent | Excellent | Bad | Bad | Bad | Bad | None | None | Excellent | Good | None | None | Bad | Bad | Bad | Bad |
| 4 | None | None | Good | Good | Bad | Bad | None | None | Excellent | Excellent | None | None | Good | Good | Bad | Bad |
| 10 | Excellent | Excellent | Excellent | Excellent | Good | Good | Bad | Bad | Excellent | Excellent | Bad | Bad | Excellent | Good | Good | Excellent |
| 18 | Excellent | Excellent | Excellent | Excellent | Excellent | Excellent | Bad | Bad | Excellent, | Excellent | Good | Good | Excellent | Excellent | Excellent | Excellent |
| 22 | Excellent | Excellent | Excellent | Excellent | Excellent | Excellent | None | None | Excellent | Excellent | Excellent | Excellent | Excellent | Excellent | Excellent | Excellent |
| 25 | Bad | Bad | None | None | None | None | Bad | Bad | None | Bad | None | Good | None | None | None | Bad |
| 30 | Bad | Bad | None | None | None | None | Bad | Bad | Excellent | Bad | Excellent | Good | None | None | Excellent | Bad |
| 37 | Bad | Bad | None | None | None | None | Bad | Bad | Good | Bad | Excellent | Good | None | None | Excellent | Bad |

### (Experiment 3 Evaluation of Relationship Between Barrier Property and Amount of MN: Transition Evaluation of Medicine to Support Using Medical Adhesive Patch Samples)

### (3-1 Preparation of Samples)

A formula of the adhesive layer of the samples of the present experiment is shown as follows.

| | |
|---|---|
| SIS | 25 parts |
| Alicyclic saturated hydrocarbon resins | 42 parts |
| Liquid paraffin | 18 parts |
| IPM | 10 parts |
| Escitalopram | 5 parts |

According to the formula described above, the escitalopram, the IPM, and the liquid paraffin were sufficiently mixed. Then, a mixed liquid formed of the SIS, the alicyclic saturated hydrocarbon resins, and toluene was added to the obtained mixture to prepare medical coating liquid. The medical coating liquid was applied to a peel-off liner (peel-off member) prepared of PET, and a solvent was dried and removed to form an adhesive layer containing a medicine. Furthermore, the adhesive layer was attached to the barrier layer side of the support film prepared with the method disclosed in 1-1 as a film of the support and a medical adhesive patch was obtained. 8 stages of the amount of MN of the support film were 1 wt%, 2 wt%, 4 wt%, 10 wt%, 18 wt%, 22 wt%, 30 wt%, and 37 wt%, and 8 types of samples of medical adhesive patches were prepared.

### (3-2 Experiment Procedure)

### a. Stability Test for Non-Elongation Time

A medical adhesive patch sample obtained by cutting to be 10 square centimeters was stored at 60°C for 1 week while exposing the adhesive layer by removing the peel-off member without performing an elongation operation. This experiment was reviewed using each of the amounts of MN.

### b. Stability Test for Elongation Time

A medical adhesive patch sample obtained by cutting to have a width of 30 mm and a length of 50 mm was stored at a room temperature for a day after removing the peel-off member and performing the elongation operation in a length direction once. 4 stages of the elongation rate were 0%, 5%, 10%, and 20%, and the 6 stages of the amount of MN were 1 wt%, 10 wt%, 18 wt%, 22 wt%, 30 wt%, and 37 wt%, for the evaluation.

### (3-3 Evaluation of Medical Transition)

In any of a and b described above, the release amounts of drug with respect to the samples after the storing period were measured. The measurement of the release amounts was performed for 4 hours under the following conditions according to rotating cylinder method (Apparatus 6 (Cylinder)) disclosed in a release test (Physical test / <724> Drug Release) of US Pharmacopeia (USP: US27-NF22).

| Test liquid | disintegration test second liquid |
|---|---|
| Liquid temperature | 32±0.5°C |
| Distance between a lower end of a cylinder and an inner bottom surface of a vessel | 12±2 mm |
| Rotation speed | 50 rpm |

The total release amounts with respect to amounts mixed to the adhesive layer were stated as release rate (%). Since a release rate in the experiment of the medical adhesive patch sample which is different from only a point in that the PET which almost does not adsorb the medicine was used as the barrier layer, was 65%, this was set as a reference, and when the release rate was decreased, it was assumed that the transition to the support or the adsorption occurred, and the evaluation was performed. The evaluation was set as three stages. Transition to the support is not recognized: Excellent, slight transition is assumed, but it does not affect the quality: Good, large amounts of transition are assumed, and it is not suitable for a medical adhesive patch: Bad.

### (3-4 Experiment Results)

The result is shown in Table 3. In the experiment of a, the transition to the support was almost not seen in the samples having an amount of equal to or more than 4%. In the experiment of b, a barrier property with no problem was reviewed, however, in the samples having an amount of MN exceeding 22%, the transition of the medicine to the support in a part of the elongated sample was seen. It was assumed that cracks are generated on the barrier layer due to the elongation operation.

In addition, in all experiments, the samples having a release rate exceeding 65% were seen; however, it is assumed to depend on the measurement error.

**[Table 3]**

| | Release amount of medicine (%) | | | | |
|---|---|---|---|---|---|
| | 60°C 1W | After elongation, a day in room temperature | | | |
| Amount of MN (wt%) | 0% | 0% | 5% | 10% | 20% |
| 1 | Bad, 56 | Excellent, 65 | Excellent, 65 | Excellent, 65 | Excellent, 65 |
| 2 | Bad, 58 | | | | |
| 4 | Good, 61 | | | | |
| 10 | Good, 63 | Excellent, 65 | Excellent, 65 | Excellent, 65 | Excellent, 65 |
| 18 | Good, 63 | Excellent, 66 | Excellent, 65 | Excellent, 65 | Excellent, 65 |
| 22 | Excellent, 67 | Excellent, 68 | Excellent, 68 | Excellent, 67 | Excellent, 66 |
| 25 | | | | | |
| 30 | Good, 64 | Excellent, 65 | Good, 64 | Good, 63 | Bad, 56 |
| 37 | Good, 63 | Excellent, 65 | Bad, 57 | Bad, 60 | Bad, 53 |

When comprehensively determining the results from Experiments 1 to 3, if the amount of MN of the barrier layer 12 is in a range of equal to or more than 2 wt% and equal to or less than 22 wt%, it was considered to be able to configure a medical adhesive patch in which a barrier property is secured to an extent to sufficiently suppress the transition of the plasticizer and the medicine to the support in any of the non-elongation time and the 20% elongation time.

### (Experiment 4 Evaluation of Relationship between Degree of Saponification of PVA and Adhesiveness of Support-Barrier Layer: Evaluation of water resistance adhesion)

### (4-1 Preparation of Sample)

The support is prepared in the same way as in Experiment 1, and the degree of saponification of PVA to be used for a barrier layer was set to have four stages of 80%, 90%, 95.5% and 98.5% (complete saponification). A barrier coating material was prepared by mixing the PVA of each degree of saponification and MN, and was applied to form a barrier layer with the same amount and method as Experiment 1, and a sample 120 of the support film was prepared. The amount of MN in the barrier layer was 10 wt%.

### (4-2 Experiment Procedure)

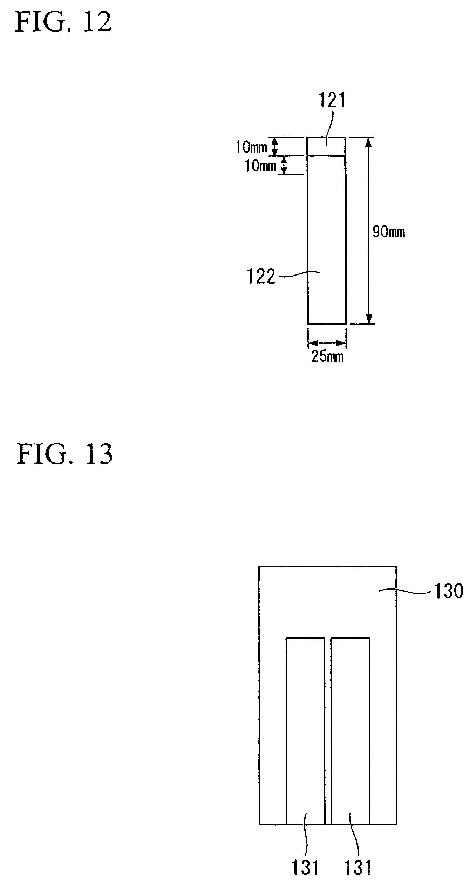

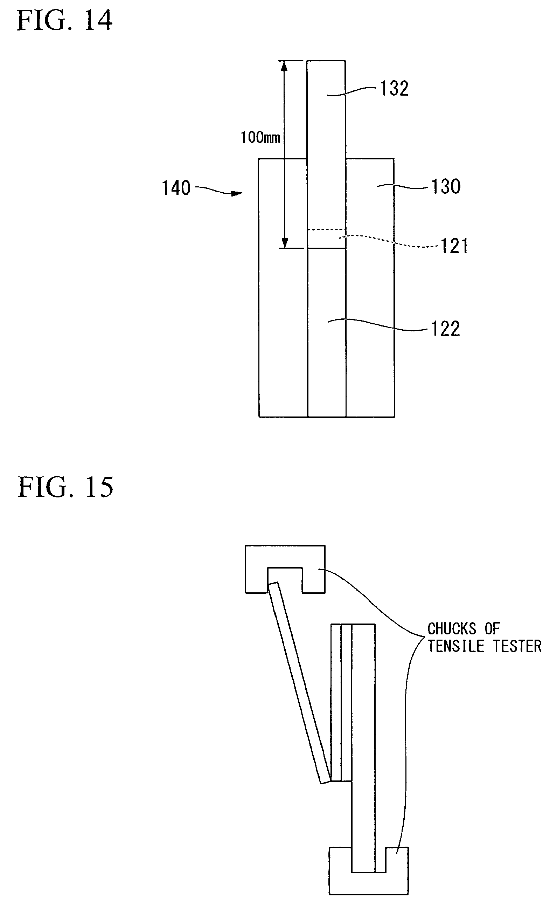

a. After cutting an adhesive tape 122 to be 30 mm X 100 mm and attaching a PET sheet 121 on which silicon is applied to one end in the longitudinal direction, the adhesive tape is attached to the barrier layer of the sample 120 as shown in FIG. 11.
b. The adhesive tape 122, the PET sheet 121 and the sample 120 were cut to have a size of 25 mm X 90 mm as shown in FIG. 12.
c. As shown in FIG. 13, two double-sided tapes 131 having a size of 25 mm X 90 mm are attached to be in parallel to each other to an acrylic plate 130, and the support side of the cut adhesive tape 122 and the double-sided tapes 131 are adhered so as to cover two double-sided tapes 131. A part of double-sided tapes 131 which protrudes in a width direction of the adhesive tape 122 is cut off to remove from the acrylic plate 130.
d. A reinforcement tape 132 having a size of 50 mm X 100 mm is prepared, and as shown in FIG. 14, the reinforcement tape 132 is attached to the end of the adhesive tape 122 which is not adhered to the sample 120 so as to interpose the PET sheet 121 in the thickness direction, to prepare an evaluation piece 140.
e. The evaluation piece 140 is dipped in hot water at 40°C and left for 30 minutes. At that time, the entire adhesive tape 122 is positioned in the water.
f. The evaluation piece 140 is picked up from the hot water after 30 minutes has passed, and is set in the tensile tester after wiping off the moisture. At that time, as shown in FIG. 15, the acrylic plate 130 is fixed to one chuck, and an end of a side which is not adhered to the PET sheet of the reinforcement tape 132 is fixed to another chuck.
g. The evaluation piece is pulled with a tension rate of 300 mm/min, and the measurement ends at the point of complete peel-off of the adhesive tape 122 from the support. An average value of tension values N of the tensile tester with a range of tension amount from 10 mm to 30 mm was set as a water resistance adhesion. Three evaluation pieces were prepared for a sample and the water resistance adhesion was evaluated.

### (4-3 Experiment Results)

The result is shown in Table 4. With the evaluation piece having the degree of saponification of PVA of equal to or less than 95.5%, the average value of the tension values N was equal to or more than 10 Newtons (N) and excellent water resistance adhesion was shown. With the evaluation piece having the degree of saponification of 98.5%, the water resistance adhesion was significantly degraded. Accordingly, in order to obtain excellent adhesiveness of the support and the barrier layer, it is considered that the degree of saponification of the water-soluble polymer is preferable to be equal to or less than 95.5%.

**[Table 4]**

| Degree of saponification of PVA | Amount of MN (wt%) | Water resistance adhesion [N / 25 mm width] | | |
|---|---|---|---|---|
| | | n = 1 | n = 2 | n = 3 |
| 80 | 10 | 13.5 | 13.1 | 17.2 |
| 90 | | 10.6 | 10.2 | 10.6 |
| 95.5 | | 14.1 | 13 | 13.5 |
| 98.5 | | 1.9 | 2.6 | 2.1 |

### (Experiment 5 Evaluation of Relationship between Degree of Saponification of PVA and Adhesiveness of Support-Barrier Layer: Evaluation with bath)

In Experiment 5, as an evaluation for performing in a closer usage environment of the medical adhesive patch, an evaluation with bath was performed.

### (5-1 Preparation of Sample)

### a. Preparation of Support Film

A support was prepared as same as Experiment 1, and a degree of saponification ofPVA to be used for a barrier layer is set to have four stages of 80%, 90%, 95.5% and 98.5% (complete saponification). A barrier coating material was prepared by mixing the PVA of each degree of saponification and MN, and was applied to form a barrier layer with the same amount and method as Experiment 1, and four types of the support film was prepared. The amount of MN of the barrier layer was 10 wt%.

### b. Preparation of Adhesive Layer

A formula of the adhesive layer of the samples of the present experiment is shown as follows.

| | |
|---|---|
| SIS | 100 parts |
| Alicyclic saturated hydrocarbon resins | 80 parts |
| Liquid paraffin | 10 parts |

According to the formula described above, the SIS, the alicyclic saturated hydrocarbon resins, and liquid paraffin were dissolved in toluene to prepare coating liquid. After coating this coating liquid on the barrier layer side of the support film described above, the barrier layer was dried and the solvent of the coating liquid removed to form an adhesive layer.

As described above, a sample having applied amount of the adhesive layer after drying of approximately 100 g/m² was obtained.

### (5-2 Experiment Procedure)

The sample (10 cm²) was attached to the upper arms of a person. Firstly, the person take a bath immediately after attaching the sample, and secondly, after the person take a bath after 24 hours from attaching the sample, the peeling-off of the support film of the adhesive patch was visually evaluated. Movement in the bath, for example, washing of the body, was maintained constant
The evaluation was performed by setting n = 2, and evaluation criteria were set as follows.
Good: Two persons did not recognize peeling-off of the support films.
Poor: One of two persons recognized peeling-off of the support films.
Bad: Two persons recognized peeling-off of the support films.

### (5-3 Experiment Results)

The results are shown in Table 5. When the degree of saponification is equal to or less than 95.5%, all test subjects recognized peeling-off of the support of the sample, however one thereof recognized peeling-off of the support with the sample used PVA having complete degree of saponification.

When comprehensively determining the results of Experiments 4 and 5, it is considered that, a suitable range of the degree of saponification of PVA which shows excellent water resistance adhesion in the medical adhesive patch 1 of the embodiment is equal to or less than 95.5%.

**[Table 5]**

| Degree of saponification of PVA (%) | Water resistance adhesion |
|---|---|
| 80 | Good |
| 90 | Good |
| 95.5 | Good |
| 98.5 | Poor |

As described above, in the medical adhesive patch 1 of the embodiment, by setting the amount of the MN of the barrier layer to be equal to or more than 2 wt% and equal to or less than 22 wt%, although the support formed of polyurethane is elongated until the elongation rate of 20%, it is possible to suitably maintain a barrier property. As a result, in any case of the non-elongation time and the elongation with the elongation rate of 20%, it is possible to obtain a medical adhesive patch which suitably maintains a barrier property, and suitably prevents transition of a plasticizer or a medicine included in a adhesive layer to a support.

Furthermore, by setting the degree of saponification of PVA of the barrier layer to be equal to or more than 70% and equal to or less than 95.5%, it is possible to obtain a medical adhesive patch which has excellent adhesiveness of a support and a barrier layer, resists in various use conditions, and contributes to maintain the quality of life (QOL) of a patient.

In addition, as shown in Table 6 described below, when a barrier property of a plasticizer in the case of fixing the amount of the MN of the barrier layer to 10 wt% and setting the degree of saponification of PVA to be 90%, 95%, and 98.5% are evaluated, it is evaluated that the degree of saponification does not significantly affect the barrier property in a range of the degree of saponification equal to or more than 90%.

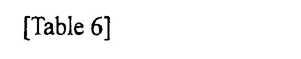

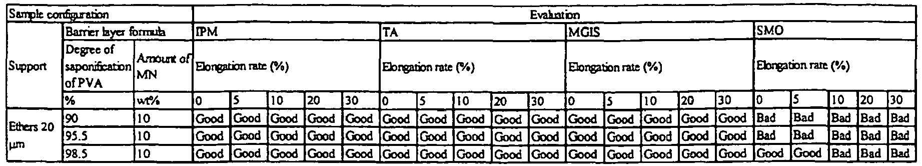

As described above, the embodiment of the present invention has been described, however, the technique scope of the present invention is not limited to the embodiment described above, and it is possible to change combinations of constituent elements of each embodiment, to add and remove various modifications to and from each constituent element in a range not departing from the purpose of the present invention.

For example, it is not essential to set the degree of saponification of PVA to the range described above in the medical adhesive patch of the present invention. Accordingly, PVA having a value of a degree of saponification out of the range described above may be used for the barrier layer in a case of being attached to a part where water resistance is almost not requested, or the like.

The present invention can be widely used for medical adhesive patches using various medicines.

## Claims

1. A medical adhesive patch comprising:
a support film comprising a barrier layer containing polyvinyl alcohol with a degree of saponification in a range equal to or more than 70% and equal to or less than 95.5% and montmorillonite, the barrier layer being laminated on one surface of a support including polyurethane; and
an adhesive layer containing a medicine, a plasticizer, and a rubber-based polymer, being laminated on a barrier layer of the support film, wherein
a percentage content of montmorillonite in the barrier layer is equal to or more than 10 percent by weight and equal to or less than 22 percent by weight, and
the plasticizer is isopropyl myristate or glyceryl monoisostearate.

## Patentansprüche

1. Medizinisches Klebepflaster umfassend:
einen Trägerfilm, welcher eine Barriereschicht umfasst, die Polyvinylalkohol mit einem Verseifungsgrad in einem Bereich gleich oder mehr als 70% und gleich oder weniger als 95,5% und Montmorillonit enthält, wobei die Barriereschicht auf einer Oberfläche eines Polyurethan einschließenden Trägers laminiert ist; und
eine Klebeschicht, welche ein Medikament, einen Weichmacher und ein gummibasiertes Polymer umfasst und welche auf einer Barriereschicht des Trägerfilms laminiert ist, wobei
ein prozentualer Anteil des Montmorillonits in der Barriereschicht gleich oder mehr als 10 Gewichtsprozent beträgt und gleich oder weniger als 22 Gewichtsprozent beträgt, und
der Weichmacher Isopropylmyristat oder Glycerolmonoisostearat ist.

## Revendications

1. Timbre adhésif médical comprenant :
un film support comprenant une couche barrière contenant de l'alcool polyvinylique présentant un degré de saponification dans un plage supérieure ou égale à 70 % et inférieure ou égale à 95,5 % et de la montmorillonite, la couche barrière étant laminée sur une surface d'un support comportant du polyuréthane ; et
une couche adhésive contenant un médicament, un plastifiant et un polymère à base de caoutchouc étant laminée sur une couche barrière du film support, dans lequel
un pourcentage de montmorillonite dans la couche barrière est supérieur ou égal à 10 pour-cent en poids et inférieur ou égal à 22 pour-cent en poids, et
le plastifiant est du myristate d'isopropyle ou du monoisostéarate de glycéryle.
